# EUROPÄISCHE PATENTSCHRIFT

(11) **EP 1 918 381 B1**
(45) Veröffentlichungstag und Bekanntmachung des Hinweises auf die Patenterteilung: **31.03.2010**
(21) Anmeldenummer: 06022740.2
(22) Anmeldetag: 31.10.2006
(51) Int. Cl.: C12P 7/08, A23C 9/142, A23C 9/144, A23C 9/146, C13K 5/00

(54) **Verfahren zur Herstellung von Ethanol aus Molke**
Process for production of ethanol from whey
Procédé de production d'éthanol à base de petit-lait

(43) Veröffentlichungstag der Anmeldung: 07.05.2008
(73) Patentinhaber: Molkerei Alois Müller GmbH & Co. KG, 86850 Aretsried (DE)
(72) Erfinder: Cloidt, Roland, 01454 Radeberg (DE); Lehmann, Hanno, 59846 Sundern (DE); Jansen, Martin, 01097 Dresden (DE)
(74) Vertreter: Leinweber & Zimmermann

(56) Entgegenhaltungen:
- US-A- 4 617 861
- US-A- 5 563 069
- M. C. DALE & M. MOELHMAN: "A low-energy continuous reactor-separator for ethanol from starch, whey permeate, permeate mother liquor, molasses or cellulosics" ENERGIE CITATIONS DATABASE, [Online] 14. April 1997 (1997-04-14), XP002429901 Gefunden im Internet: URL:http://www.osti.gov/energycitations/se rvlets/purl/469182-RsHsi0/webviewable/4691 82.pdf> [gefunden am 2007-04-17]
- CARIC MARIJANA: "Concentrated and dried dairy products , Lactose" CONCENTRATED AND DRIED DAIRY PRODUCTS, VCH PUBLISHERS, NEW YORK, NY, US, 1991, Seiten 227-234, XP002410105
- GONZALEZ SISO M I: "The biotechnological utilization of cheese whey: A review" BIORESOURCE TECHNOLOGY, Bd. 57, Nr. 1, 1996, Seiten 1-11, XP002429847 ISSN: 0960-8524

## Beschreibung

Die Erfindung betrifft ein Verfahren zur Herstellung von Ethanol aus Molke.

Bei der Herstellung von Käse aus Milch oder Kasein aus Magermilch entsteht als Koppelprodukt Molke. Molke besteht im wesentlichen aus Eiweißen, Lactose und Mineralien.

Zur wirtschaftlichen Verwertung von Molke ist es bekannt, diese in Ethanol umzuwandeln. Zu diesem Zweck wird ein Verfahren angewendet, bei dem der Molke durch Ultrafiltration Eiweiß entzogen wird, und gegebenenfalls dem so gewonnenen Permeat durch ein Membrantrennverfahren oder eine Elektrodialyse oder ein lonenaustauschverfahren Wasser entzogen wird. Das auf diese Weise gewonnene Lactosekonzentrat (UF Permeat) wird durch Zugabe von Hefen fermentiert, das fermentierte Substrat wird destilliert, und der bei der Destillation erhaltene Rohalkohol kann in einer nachgeschalteten Absolutierung entwässert werden. Mit diesem bekannten Verfahren können aus 100 kg Trockenmasse (Lactose) enteiweißter Molke ca. 54 Liter Ethanol hergestellt werden, was derzeit einem Marktwert von ca. 32,-- EUR entspricht.

Eine höhere Wertschöpfung läßt sich erzielen, wenn die enteiweißte Molke nicht in Ethanol umgewandelt wird, sondern zuerst Lactose aus diesem Rohstoff gewonnen wird. Zu diesem Zweck wird ein Verfahren angewendet, bei dem Molke durch Ultrafiltration Eiweiß entzogen wird, dem so gewonnenen Permeat durch ein Membrantrennverfahren oder durch Elektrodialyse / lonenaustausch Wasser entzogen wird, die Konzentration des so gewonnenen Lactosekonzentrates durch Eindampfen auf mindestens 60% Trockenmasse erhöht wird, die in dem Konzentrat enthaltene Lactose auskristallisiert wird, das kristallisierte Lactosekonzentrat in einer Wasch- und Konzentrationsstufe weiter aufkonzentriert und gereinigt wird und die dabei entstehende Melasse abgeschieden wird, während das verbleibende Lactosekonzentrat getrocknet wird. Mit diesem bekannten Verfahren können aus 100 kg Trockenmasse Lactose ca. 58 kg Lactosepulver hergestellt werden, was derzeit einem Marktwert von ca. 40,-- EUR entspricht.

Bei der Umwandlung von Molke in Lactosepulver ist die Wertschöpfung demzufolge größer als bei der Umwandlung in Ethanol. Die bei der Herstellung von Lactosepulver als Koppelprodukt entstehende Melasse hat praktisch keinen Marktwert und wird zum Teil als Viehfutter verwendet bzw. auch über "Landversprühung" entsorgt. Bei dieser Melasse handelt es sich um ein teilentzuckertes Permeat, das ca. 20% Trockenmasse enthält, davon 60 bis 75% Lactose, 15 bis 25% Mineralien, die sogenannte Asche, und 5 bis 15% Proteine sowie in geringen Mengen sonstige Molkeninhaltsstoffe. Trotz des hohen Anteils an Lactose ist es bis heute nicht gelungen, eine wirtschaftliche Verwertung der Melasse technisch umzusetzen.

In der Literaturstelle "A Low-energy Continuous Reactor-Separator for Ethanol from Starch, Whey Permeate, Permeate Mother Liquor, Molasses or Cellulosics" XP002429901 NIST/DOE Energy-Related Invention Project Final Report Project DE-FG01-94CE 15594 4/1/94 through 2/28/97 wird vorgeschlagen, Molkepermeat durch Eindampfen auf 50 % Trockenmasse aufzukonzentrieren, aus dem Konzentrat die Lactose auszukristallisieren und danach durch Zentrifugieren eine Auftrennung in Lactose und Melasse vorzunehmen. Diese Melasse wird sodann in einem Fermentationsreaktor durch Hefezugabe, insbesondere vom Stamm Kluyveromyces marxianus, fermentiert und der entstehende Alkohol durch extraktive Destillation gewonnen.

Der Erfindung liegt die Aufgabe zugrunde, das bekannte Verfahren dahingehend weiterzubilden, daß eine wirtschaftliche Verwertung der als Koppelprodukt entstehenden Melasse möglich ist.

Die Lösung dieser Aufgabe ist im Patentanspruch 1 angegeben.

Mit dem erfindungsgemäßen Verfahren können aus 100 kg Trockenmasse Lactose zusätzlich zu den 58 kg Lactosepulver noch 17 Liter Ethanol gewonnen werden, was einem derzeitigen Marktwert von ca. 10 EUR entspricht. Die gesamte Wertschöpfung beträgt somit 50 EUR pro 100 kg Trockenmasse (Lactose) enteiweißter Molke.

Die veröffentlichte Patentanmeldung DE 4113836 A1 offenbart ein Verfahren zur Aufarbeitung von Süßmolkenpermeat unter Gewinnung von hochreiner, weißer Lactose und weiteren industriell verwertbaren Feststoffgemischen, welches dadurch gekennzeichnet ist, daß man ein bis auf einen Trockensubstanzgehalt von 30 bis 60 Gew.-% eingeengtes und auf einen pH-Wert von 6,8 bis 7,2 eingestelltes Süßmolkenpermeat zur Ausfällung eines Calciumphosphat-angereicherten Lactose/Protein/Mineralsalz-Feststoffgemisches auf eine unterhalb der Siedetemperatur liegende Temperatur von mindestens 80°C während einer zur Ausfällung des bei dieser Temperatur fällbaren Anteils des Gesamt-Calciumphosphatgehaltes des Süßmolkenpermeates ausreichenden Zeitdauer erhitzt, das ausgefällte Feststoffgemisch abtrennt, das nach der Abtrennung des Feststoffgemisches verbleibende Süßmolkenpermeat für eine zur Entfärbung ausreichende Zeitdauer mit Aktivkohle kontaktiert und sodann von der Aktivkohle abtrennt, das von der Aktivkohle abgetrennte entfärbte Süßmolkenpermeat auf einen Trockensubstanzgehalt von 60 bis 80 Gew.-% einengt und die Lactose auskristallisieren läßt, die auskristallisierte Lactose von einer verbleibenden Mutterlauge abtrennt und aus der Mutterlauge durch Mikrofiltration ein weiteres Feststoffgemisch abtrennt.

Wegen des hohen Lactosegehalts der Melasse entsteht bei der Fermentierung bzw. Vergärung ein Produkt mit einem verhältnismäßig hohen Alkoholgehalt. Wegen des hohen Alkoholgehalts sind nicht alle der an sich zur Fermentierung von Lactose geeigneten Hefen brauchbar. Es können aber gute Ergebnisse erzielt werden, wenn der Melasse Hefen der Gattung Klayveromyces beigegeben werden.

Zur weiteren Steigerung der Wirtschaftlichkeit des erfindungsgemäßen Verfahrens ist vorgesehen, daß die Hefe von dem fermentierten Substrat getrennt und aufkonzentriert wird, daß ein Teil der hochkonzentrierten Hefe zur Fermentierung der Melasse rückgeführt wird, und daß die verbleibende Klarphase des fermentierten Substrates destilliert wird.

Ein Ausführungsbeispiel der Erfindung wird nachfolgend näher erläutert. Es zeigt:

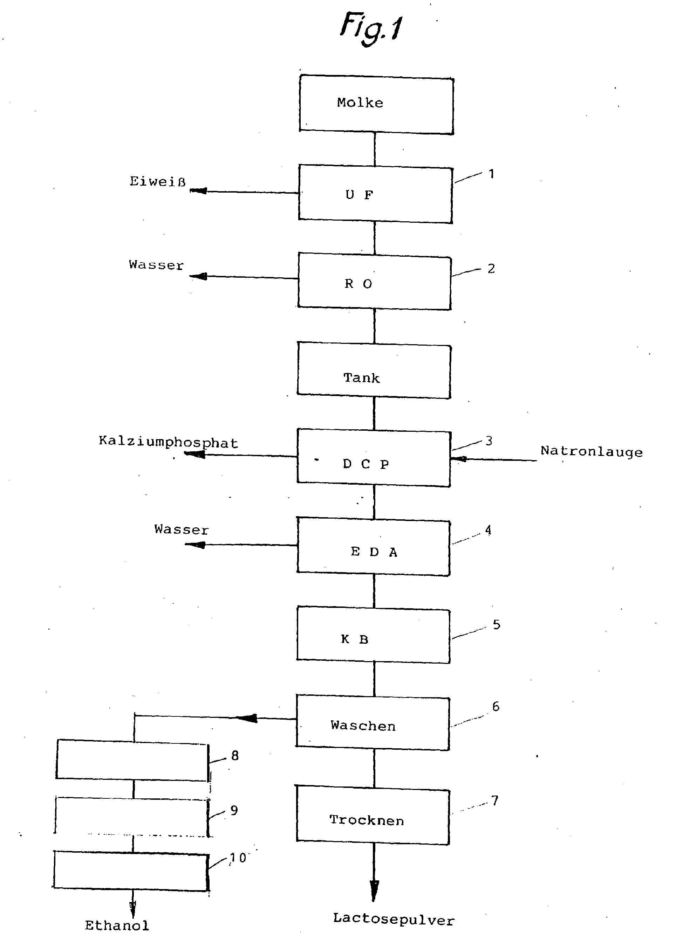

Fig. 1 eine schematische Darstellung der einzelnen Schritte eines Verfahrens zur Herstellung von Lactose und Ethanol aus Molke, die entsprechend ihrer zeitlichen Reihenfolge fortlaufend numeriert sind, und
Fig. 2 eine ausführlichere Darstellung der einzelnen Schritte zur Herstellung von Ethanol aus der bei dem Verfahren nach Fig. 1 anfallenden Melasse.

Der als Ausgangsstoff dienenden Molke, die beispielsweise bei der Herstellung von Käse anfällt, wird in einem ersten Verfahrensschritt durch Ultrafiltration Eiweiß entzogen. Dem so gewonnenen Permeat wird sodann in einem zweiten Verfahrensschritt durch ein Membrantrennverfahren oder eine Elektrodialyse/lonenaustauscher und/oder ein Eindampfverfahren Wasser entzogen. Das dadurch gewonnene Retentat kann in einem Tank zwischengelagert werden. In einem dritten Verfahrensschritt wird der pH-Wert im Lactosekonzentrat durch Zugabe von Natronlauge (NaOH) eingestellt, das so behandelte Lactosekonzentrat erhitzt und heißgehalten, wodurch das in dem Lactosekonzentrat enthaltene Kalziumphosphat (Ca₂(PO₃)⁴) ausgefällt wird. Der dritte Verfahrensschritt umfaßt auch das mechanische Abtrennen des ausgefällten Kalziumphosphats. Die Ausfällung und die mechanische Abtrennung von Kalziumphosphat ist aus zwei Gründen vorgesehen. Einerseits wird die weitere Verarbeitung des Lactosekonzentrates durch das Kalzium beeinträchtigt und andererseits ist Kalziumphosphat bei entsprechender Reinheit ein Handelsprodukt.

In einem vierten Verfahrensschritt wird die Konzentration des so behandelten Konzentrates durch Eindampfen auf mindestens 60% Trockenmasse erhöht. Die in dem Konzentrat enthaltene Lactose wird anschließend in einem fünften Verfahrensschritt kristallisiert. Die kristalline Lactose wird in einem sechsten Verfahrensschritt in einer mehrstufigen Wasch - und Dekantier Anlage gewaschen und weiter aufkonzentriert. Das gewaschene Lactosekonzentrat mit einem Gehalt von >90% Trockenmasse wird sodann in einem siebten Verfahrensschritt getrocknet, so daß ein Lactosepulver mit >99,7% Trockenmasse erhalten wird.

Die beim Waschen und Konzentrieren des Lactosekonzentrats entstehende Melasse enthält ca. 20% Trockenmasse, die ihrerseits aus 60 bis 75% Lactose besteht. In einem achten Verfahrensschritt wird die Melasse fermentiert. Das dabei entstehende Produkt, die sogenannte Maische, wird in einem neunten Verfahrensschritt destilliert. Der durch die Destillation gewonnene Rohalkohol mit einem Alkoholgehalt von 80 bis 85% wird in einem zehnten Verfahrensschritt absolutiert bzw. dehydriert, so daß ein Endprodukt erhalten wird, das mehr als 99,5% Ethanol enthält.

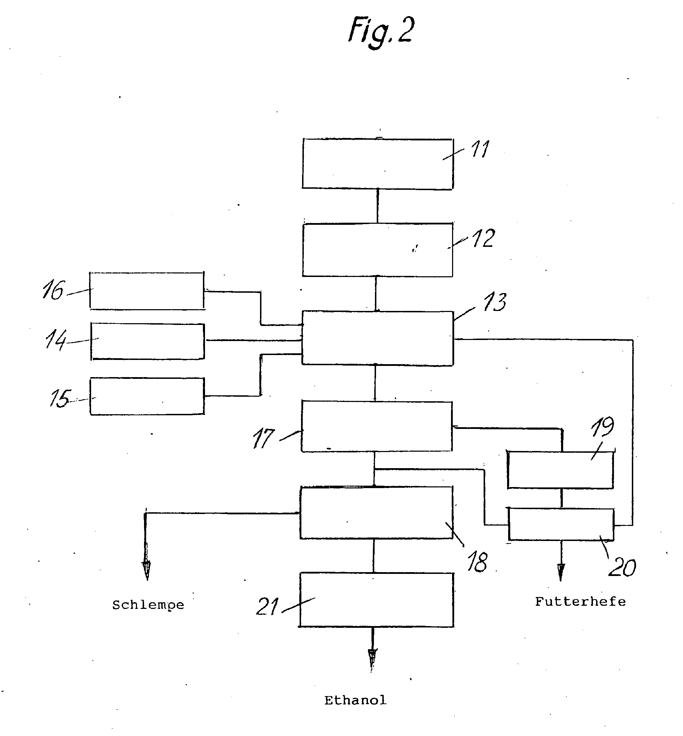

In Fig. 2 sind die bei der Verarbeitung der beim Waschen und Dekantieren des Lactosekonzentrats anfallenden Melasse durchgeführten Schritte näher erläutert.

In einem Tank 11 wird Melasse bereitgestellt mit 20% Trockenmasse, davon 60 bis 75% Lactose. Von dem Tank 11 gelangt die Melasse in einen Erhitzer 12, wo sie zur Abtötung von Keimen auf ca. 85°C erhitzt wird. Nach der Erhitzung wird die Melasse auf die benötigte Fermentationstemperatur von 25 bis 35°C abgekühlt und in einen oder mehrere Fermentationstanks 13 überführt. Im Verlauf bzw. nach der Befüllung des Fermentationstanks 13 wird aus einem Kulturtank 14 die zur Fermentation/Gärung der Melasse erforderliche Hefe zugeführt. Zur Einstellung eines geeigneten p_{H} Wertes von 4 bis 6 kann Natronlauge bzw. Säure aus einem Vorlagebehälter 15 zugeführt werden. Während des Aufschlusses und/oder der Fermentierung der Lactose wird das Substrat umgewälzt und über einen Kühlvorgang auf Fermentationstemperatur gehalten. Die Umwälzung stabilisiert gleichzeitig den Fermentationsprozeß (Temperatur - Wirkoptimum der Hefen) und unterstützt den Austrag des bei der Fermentation entstehenden Kohlendioxids. Wegen des hohen Lactosegehaltes der Melasse entsteht bei deren Fermentation ein verhältnismäßig hoher Alkoholgehalt, wodurch die Wirksamkeit gängiger Hefen beeinträchtigt wird. Es hat sich aber gezeigt, daß Hefen der Gattung Klayveromyces für die Fermentation der Melasse bestens geeignet sind. Die Verweildauer der Melasse in den Fermentationstanks 13 beträgt je nach Lactosekonzentration bis zu 100 Stunden.

Das bei der Fermentation entstehende Kohlendioxid wird gesammelt und gewaschen, um die bei der Verflüchtigung mitgerissenen Alkoholbestandteile zurückzugewinnen. Anschließend wird das Kohlendioxid in einer Verflüssigungsanlage 16 verflüssigt bzw. ganz oder zum Teil über Dach emitiert.

Nach abgeschlossener Fermentierung steht das fermentierte Substrat für die Weiterverarbeitung bereit. Das fermentierte Substrat wird in einem Düsenseparator 17 von Hefen getrennt. Das fermentierte und hefenarme Substrat wird sodann einer Destille 18 zugeführt. Das in dem Düsenseparator 17 separierte Hefenkonzentrat wird in einer Wascheinrichtung 19 mit Wasser versetzt und anschließend in einem zweiten Düsenseparator 20 aufkonzentriert. Die in dem zweiten Düsenseparator 20 abgetrennte Klarphase wird dem der Destille 18 zulaufenden Produkt beigemischt um Restalkohole zurück zu gewinnen. Das in dem zweiten Düsenseparator 20 gewonnene Hefenkonzentrat wird bis zu 50% in den oder die Fermentationstanks 13 rückgeführt. Das nicht rückgeführte Hefenkonzentrat kann als Futterhefe bereitgestellt werden.

Das der Destille 18 zugeführte fermentierte Substrat sowie die mit Alkohol versetzte Klarphase des zweiten Düsenseparators 20 wird auf ca. 80 bis 95%-igen Rohalkohol destilliert, rektifiziert und einer direkt nachgeschalteten Dehydrationseinrichtung 21 zugeführt. In der Dehydrationseinrichtung 21 wird der (Roh) Alkohol bis auf eine Reinheit von mindestens 99,7% entwässert, so daß ein Handelsfähiger Bio- Ethanol / Alkohol erhalten wird.

Die bei der Destillation des fermentierten Substrats anfallende Schlempe kann zur weiteren Verwendung durch Eindampfen auf 20 - 30% Trockenmasse konzentriert werden.

## Patentansprüche

1. Verfahren zur Herstellung von Ethanol aus Molke, bei dem der Molke durch Ultrafiltration Eiweiß entzogen wird, dem so gewonnenen Permeat durch ein Membrantrennverfahren oder eine Elektrodialyse oder ein lonenaustauschverfahren Wasser entzogen wird, in dem dadurch gewonnenen Retentat der pH-Wert durch Zugabe von Natronlauge eingestellt, das so behandelte Lactosekonzentrat erhitzt und heißgehalten und dadurch das in dem Lactosekonzentrat enthaltene Kalziumphosphat ausgefällt wird, das ausgefällte Kalziumphosphat mechanisch abgetrennt wird, die Konzentration des so gewonnenen Lactosekonzentrates durch Eindampfen auf mindestens 60 % Trockenmasse erhöht wird, die in dem Konzentrat enthaltene Lactose auskristallisiert wird, das kristallisierte Lactosekonzentrat in einer Wasch- und Konzentrationsstufe zerlegt wird in hochkonzentrierte Lactose und Melasse, die Melasse durch Zugabe von Hefen fermentiert wird, das fermentierte Substrat destilliert, rektifiziert wird und der bei der Destillation/Rektifikation erhaltene (Roh)Alkohol in einer nachgeschalteten Absolutierung entwässert wird.

2. Verfahren nach Anspruch 1, **dadurch gekennzeichnet, daß** der Melasse Hefen der Gattung Kluyveromyces beigegeben werden.

3. Verfahren nach Anspruch 2, **dadurch gekennzeichnet, daß** die Hefe von dem fermentierten Substrat getrennt und aufkonzentriert wird, daß ein Teil der hochkonzentrierten Hefe zur Fermentierung der Melasse rückgeführt wird, und daß die verbleibende Klarphase des fermentierten Substrates destilliert wird.

## Claims

1. A method for the production of ethanol from whey wherein albumen is extracted from the whey by ultrafiltration, water is withdrawn from the permeate thus obtained by means of a membrane separation process or electrodialysis or an ion exchange process, in the retentate obtained in this way the pH value is adjusted by adding caustic soda, the lactose concentrate treated in this way is heated and kept hot, and in this way the calcium phosphate contained in the lactose concentrate is precipitated, the precipitated calcium phosphate is mechanically separated out, the concentration of the lactose concentrate obtained in this way is increased to at least 60 % dry mass by evaporating, the lactose contained in the concentrate is crystallised out, the crystallised lactose concentrate is decomposed into highly concentrated lactose and molasses in a washing and concentration stage, the molasses are fermented by adding yeasts, the fermented substrate is distilled, rectificated, and the (raw) alcohol obtained by the distillation/rectification is dehyrdated in a downstream dehydration.

2. The method according to Claim 1, **characterised in that** yeasts of the Kluyveromyees genus are added to the molasses.

3. The method according to Claim 2, **characterised in that** the yeast is separated from the fermented substrate and concentrated, that part of the highly concentrated yeast is passed back to the molasses for fermentation, and that the remaining clear phase of the fermented substrate is distilled.

## Revendications

1. Procédé de préparation d'éthanol à partir de lactosérum, dans lequel on élimine les protéines du lactosérum par ultrafiltration, on élimine l'eau du perméat ainsi obtenu selon un procédé de séparation sur membrane, un procédé par échange d'ions ou par électrodialyse, on ajuste le pH du rétentat ainsi obtenu par ajout de lessive de soude, on chauffe le concentré de lactose ainsi traité et on maintient une température élevée qui entraîne une précipitation du phosphate de calcium contenu dans le concentré de lactose, on sépare le phosphate de calcium précipité par voie mécanique, on accroît la concentration du concentré de lactose ainsi produit par évaporation pour obtenir au moins 60 % de masse sèche, on procède à la cristallisation du lactose contenu dans le concentré, on décompose le concentré de lactose cristallisé en lactose hautement concentré et en mélasse au cours d'une étape de lavage et de concentration, on fait fermenter la mélasse par ajout de levures, on distille le substrat fermenté, on le rectifie et on déshydrate l'alcool (brut), obtenu au cours de la distillation/rectification, lors d'un traitement aval visant à obtenir un alcool absolu.

2. Procédé selon la revendication 1, **caractérisé en ce que** l'on ajoute à la mélasse des levures du genre *Kluyveromyces.*

3. Procédé selon la revendication 2, **caractérisé en ce que** l'on sépare la levure du substrat fermenté et on la concentre, **en ce qu'**une partie de la levure hautement concentrée est recyclée vers la fermentation de la mélasse et **en ce que** l'on distille la phase clarifiée résiduelle du substrat fermenté.
